# EUROPEAN PATENT SPECIFICATION

(11) **EP 4 230 246 B1**
(45) Date of publication and mention of the grant of the patent: **22.01.2025**
(21) Application number: 21896669.5
(22) Date of filing: 22.10.2021
(51) Int. Cl.: A61M 16/16

(54) **WATER TANK INSTALLATION STRUCTURE AND VENTILATION THERAPY DEVICE**
WASSERTANKINSTALLATIONSSTRUKTUR UND BEATMUNGSTHERAPIEVORRICHTUNG
STRUCTURE D'INSTALLATION DE RÉSERVOIR D'EAU ET DISPOSITIF DE THÉRAPIE DE VENTILATION

(30) Priority: 25.11.2020 CN 202011342914
(43) Date of publication of application: 23.08.2023
(73) Proprietor: BMC (Tianjin) Medical Co., Ltd., Tianjin 301700 (CN)
(72) Inventor: LIU, Lijun, Beijing 100041 (CN); ZHUANG, Zhi, Beijing 100041 (CN)
(74) Representative: Grünecker Patent- und Rechtsanwälte PartG mbB
(86) International application number: PCT/CN2021/125729
(87) International publication number: WO 2022/111167

(56) References cited:
- EP-A2- 1 479 404
- WO-A1-2004/026382
- WO-A1-2016/097928
- WO-A1-2018/071812
- WO-A1-2019/189126
- CN-A- 1 939 549
- CN-A- 111 033 067
- CN-A- 112 472 937
- CN-U- 206 959 232
- CN-U- 209 237 088
- CN-U- 211 357 307
- CN-U- 211 526 629
- CN-U- 215 083 637
- US-A- 6 098 963
- US-B1- 6 256 454
- US-B1- 7 327 949

## Description

### FIELD

The present disclosure relates to the field of ventilation therapy, in particular to a water tank installation structure and a ventilation therapy device including the water tank installation structure.

### BACKGROUND

A ventilation therapy device, such as a ventilator, usually comprises a main machine, a humidifier, and a respiratory mask, wherein an inhalable air generated by the main machine is warmed and humidified by the humidifier, and then is supplied to the respiratory mask for the patient to inhale. By warming and humidifying the inhalable air with the humidifier, possible secondary infection of the respiratory tract and irritation on the cardiopulmonary system of the patient incurred by mechanical ventilation can be prevented or reduced, the pulmonary alveoli can be kept in a humid state, the activity of the pulmonary alveoli can be enhanced, the air exchange can be promoted, the consumption of the heat and the moisture content in the respiratory tract can be reduced, sputum scabs can be prevented in the respiratory tract, the viscosity of the secreta can be decreased, sputum excretion can be promoted, respiratory tract clogging can be prevented, and the compliance of the patient to the mechanical ventilation can be improved.

In the prior art, a humidifier is usually mounted in either of the two following ways: in the first way, the humidifier is arranged separately from the main machine, has an openable shell and a cavity in the shell, and a water tank is mounted in the cavity via an installation structure; in a second way, the humidifier is integrated with the main machine, i.e., the shell of the humidifier is integrated on the main machine, and a water tank is mounted in the main machine via an installation structure.

CN 211526629 U discloses a portable humidifying device comprising a body and a water tank, wherein the body is arranged above the water tank, and a humidifying device is arranged in the body, and wherein the inner wall of the outer cover of the body is provided with a screw button, and the outer wall of the water tank is provided with a screw button groove matched with the screw button. The outer cover and the water tank form a rotating screw button connection through the screw button and the screw button groove.

US 6098963 A discloses a fill-in-place humidifier comprising a tank that can be rotated 180 degrees between a filling position and an operating position, and a can that can be removed from the tank for filling in the filling position, and can be replaced when filling is complete, wherein the cap bears a valve that automatically opens when the tank is rotated into its operative positions.

US 6256454 B1 discloses a humidifier comprising an infant incubator that has a housing and a reservoir, wherein the reservoir is pivotally affixed to the housing it can be tilted from its upright position where it actively provides the heated water vapor to the incubator and a tilted position where water can be added to the reservoir.

WO 2016097928 A1 discloses a system configured to prevent damage from liquid spills in a humidified pressure support therapy device, wherein the system decouples a humidification chamber in a humidifier from a pressure generator of the pressure support therapy device when a cover of the humidifier is opened.

However, in either of the above ways, the water tank installation structure is complex, and the fitting relation is cumbersome. During use, the water tank has to be taken out for water replenishment frequently; however, the connection of the water tank to the main machine or shell is complex, and complex operations, such as cover opening and unlocking, etc. are required to take out the water tank, resulting in frequent operations of the patient and poor usability of the water tank.

### SUMMARY

In view of the above problems, the present disclosure provides a water tank installation structure and a ventilation therapy device including the water tank installation structure, to simplify the installation of a water tank and facilitate water replenishment into the water tank.

To attain the above object, in an aspect, the present disclosure provides a water tank installation structure for a ventilation therapy device, which comprises a water tank, an installation space, and a connection structure, wherein the water tank is configured to fit into and out of the installation space in a rotational manner, the connection structure is configured to connect the water tank to the installation space when the water tank is rotated into the installation space and release the connection of the water tank from the installation space when the water tank is detached from the installation space.

Further, the water tank installation structure comprises a first bottom wall and a first side wall that are used to define the installation space, the water tank comprises a liquid storage cavity, and a second bottom wall and a second side wall that are used to define the liquid storage cavity; the second bottom wall corresponds to the first bottom wall and the second side wall corresponds to the first side wall when the water tank is mounted into the installation space.

Furthermore, the first bottom wall comprises a downwardly recessed arc-shaped area, the second bottom wall forms a downwardly protruding arc-shape that matches the arc-shaped area.

Optionally, the installation space has a top opening and a side opening, which are used for installation the water tank into the installation space.

Optionally, the connection structure comprises a first connection component and a second connection component that are fitted with each other, the first connection component is arranged on one of the first side wall and the second side wall, and the second connection component is arranged on the other of the first side wall and the second side wall.

Optionally, the first connection component is on an upper part of the first side wall or the second side wall, and the second connection component is on an upper part of the first side wall or the second side wall.

Optionally, the first connection component is arranged on the first side wall, and the second connection component is arranged on the second side wall.

Optionally, the first connection component comprises a recessed cavity outwardly recessed from an inner wall surface of the first side wall and a snap-fit groove arranged on an inner surface of the recessed cavity, the second connection component comprises a boss outwardly protruding from an outer wall surface of the second side wall and a snap-fit piece arranged on the boss, the boss matches the recessed cavity, the snap-fit piece matches the snap-fit groove, the snap-fit piece is configured to be snap-fitted in the snap-fit groove when the boss is received in the recessed cavity, and can be detached from the snap-fit groove when the boss is subjected to an outwardly pulling force.

Optionally, the second connection component comprises a compression spring, the boss has a cavity therein for accommodating the compression spring and the snap-fit piece, the boss has a hole in communication with the cavity, the snap-fit piece is configured to at least partially extend out of the hole under an abutting action of the compression spring, and can retract into the cavity by compress the compression spring under pressure.

Optionally, the size of the hole is smaller than the size of the snap-fit piece.

Optionally, the snap-fit piece is a sphere, and the snap-fit groove is a hemispherical groove.

Optionally, the arc-shaped area is a heating area for heating the water tank.

Optionally, the water tank installation structure comprises a first air inlet tube, a first air outlet tube, a second air inlet tube and a second air outlet tube, the first air inlet tube is coaxially inserted in the first air outlet tube and spaced from the first air outlet tube by certain radial spacing to form a first air outflow channel, and one end port of the first air inlet tube and the first air outlet tube is located on the first side wall; the second air inlet tube is coaxially inserted in the second air outlet tube and spaced from the second air outlet tube by certain radial spacing to form a second air outflow channel, one end port of the second air inlet tube and the second air outlet tube is located on the second side wall, and the other end port of the second air inlet tube and the second air outlet tube extends into the liquid storage cavity, and the one end port of the first air inlet tube and the first air outlet tube is communicated with the one end port of the second air inlet tube and the second air outlet tube in a hermetically sealed manner via a sealing element.

Optionally, the one end port of the second air inlet tube and the second air outlet tube is on the upper part of the second side wall.

Optionally, the length of the second air inlet tube is greater than the length of the second air outlet tube, the other end port of the second air inlet tube is formed as an inclined port, a wall of the water tank opposite to the inclined port is formed as an inclined wall, so that the air from the inclined port is deflected toward a lower middle area of the liquid storage cavity.

In another aspect, the present disclosure provides a ventilation therapy device, which comprises a main machine and the water tank installation structure described above, wherein the installation space is arranged on the main machine.

With the technical scheme, in the water tank installation structure in the present disclosure, the water tank is configured to fit into and out of the installation space in a rotational manner, the connection structure is configured to connect the water tank to the installation space when the water tank is rotated into the installation space and release the connection of the water tank from the installation space when the water tank is detached from the installation space; thus, the water tank can be mounted and removed more conveniently while ensuring that the water tank is mounted in the installation space reliably; to replenish water, the water tank may be removed from the installation space by rotation to prevent water leakage into the installation space, thereby ensure the high efficiency and safety of water replenishment.

Other features and advantages of the present disclosure will be further detailed in the following embodiments.

### BRIEF DESCRIPTION OF THE DRAWINGS

The accompanying drawings are provided herein to facilitate further understanding on the present disclosure and constitute a part of this document. They are used in conjunction with the following embodiments to explain the present disclosure, but are not intended to constitute any limitation to the present disclosure. In the figures:
Fig. 1 is a schematic structural diagram of an embodiment of the ventilation therapy device in the present disclosure, which only shows the main machine and the water tank;
Fig. 2 is an exploded view of the ventilation therapy device in Fig. 1;
Fig. 3 is an isometric view of the water tank in Fig. 2 viewed from another viewing angle;
Fig. 4 is a transverse sectional view of the ventilation therapy device in Fig. 1;
Fig. 5 is a longitudinal sectional view of the ventilation therapy device in Fig. 1;
Fig. 6 is a schematic structural diagram of the main machine in Fig. 5;
Fig. 7 is a schematic structural diagram of the water tank in Fig. 5;
Fig. 8 is a schematic diagram of retraction of the snap-fit piece in Fig. 7 into the cavity of the boss;
Fig. 9 is another longitudinal sectional view of the ventilation therapy device in Fig. 1, illustrating the air flow direction;
Fig. 10 is a schematic structural diagram of the main machine in Fig. 9;
Fig. 11 is a schematic structural diagram of the water tank in Fig. 9;
Fig. 12 is a schematic structural diagram of the sealing element in Fig. 2.

### Reference Numbers

10 - water tank, 11 - liquid storage cavity, 12 - second bottom wall, 13 - second side wall, 131 - boss, 132 - cavity, 133 - hole, 134 - snap-fit piece, 135 - compression spring, 14 - second air inlet tube, 141 - inclined port, 15 - second air outlet tube, 16 - inclined wall, 20 - main machine, 21 - installation space, 211 - top opening, 212 - side opening, 22 - first bottom wall, 221 - arc-shaped area, 23 - first side wall, 231 - recessed cavity, 232 - snap-fit groove, 233 - guiding bevel, 24 - first air inlet tube, 25 - first air outlet tube, 26 - sealing element, 261 - inner ring, 262 - outer ring, 263 - connection rib.

### DETAILED DESCRIPTION

Some embodiments of the present disclosure will be detailed below with reference to the accompanying drawings. It should be understood that the embodiments described herein are only provided to describe and explain the present disclosure, but are not intended to constitute any limitation to the present disclosure.

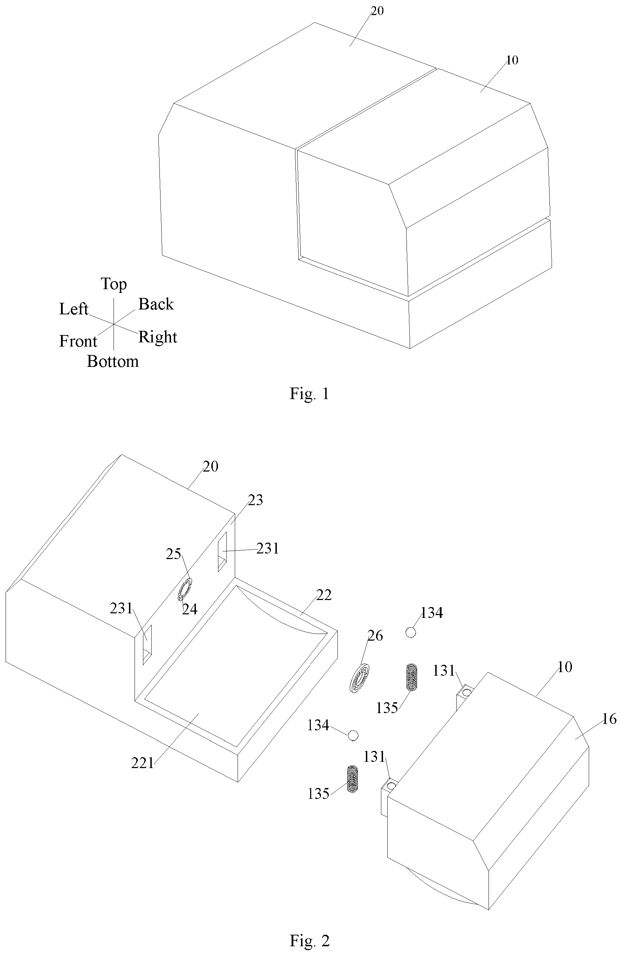

Unless otherwise specified in the present disclosure, the terms that denotes the directions or orientations, such as "top", "bottom", "front", "back", "left", "right", etc., refer to the directions or orientations as indicated in Fig. 1; "inside" and "outside" usually refers to inside and outside with respect to the outlines of the components.

In an aspect, the present disclosure provides a water tank installation structure, which comprises a water tank 10, an installation space 21, and a connection structure, wherein the water tank 10 is configured to fit into and out of the installation space 21 in a rotational manner, the connection structure is configured to connect the water tank 10 to the installation space 21 when the water tank 10 is rotated into the installation space 21, and release the connection of the water tank 10 from the installation space 21 when the water tank 10 is detached from the installation space 21.

With the technical scheme, in the water tank installation structure in the present disclosure, the water tank 10 is configured to fit into and out of the installation space 21 in a rotational manner, the connection structure is configured to connect the water tank 10 to the installation space 21 when the water tank 10 is rotated into the installation space 21 and release the connection of the water tank 10 from the installation space 21 when the water tank 10 is detached from the installation space 21; thus, the water tank 10 can be mounted and removed more conveniently while ensuring that the water tank 10 is mounted in the installation space 21 reliably; to replenish water, the water tank 10 may be removed from the installation space 21 by rotation to prevent water leakage into the installation space 21, thereby ensure the high efficiency and safety of water replenishment.

The installation space 21 has a top opening 211 and a side opening 212, which are used for mounting the water tank 10 into the installation space 21. Thus, the water tank 10 can be mounted flexibly, and is in an exposed state after it is mounted, so that water replenishment into the water tank 10 can be carried out conveniently.

During the mounting, the water tank 10 may be rotated from the top opening 211 and the side opening 212 into the installation space 21, while the water tank 10 is connected by the connection structure to the installation space 21. To replenish water into the water tank 10, it is unnecessary to remove the water tank 10 and directly add water through the exposed water inlet located on the top or side of the water tank 10; alternatively, the water tank 10 may be removed from the installation space 21 by rotation and then water can be replenished into the water tank 10. Thus, the mounting of the water tank can be greatly simplified, water can be replenished into the water tank conveniently, and the connection structure is available in a wide range of choice, which is to say, an appropriate detachable connection structure may be used.

It should be noted that the water tank 10 and the installation space 21 may be in any appropriate shape, and the side opening 212 may be arranged appropriately depending on the shape of the installation space 21. For example, in the embodiment shown in Figs. 1 - 11, the installation space 21 and the water tank 10 are generally square, and the side opening 212 covers the front side, back side and right side of the installation space 21. In other embodiments, the side opening 212 may at least cover the right side of the installation space 21, so as to provide an installation passage for the water tank 10 while the front side and/or the back side of the installation space 21 can limit the position of the water tank 10 to some extent.

In the present disclosure, as shown in Figs. 1, 2 and 5, the water tank installation structure further comprises a first bottom wall 22 and a first side wall 23 that are used to define the installation space 21, the water tank 10 comprises a liquid storage cavity 11, and a second bottom wall 12 and a second side wall 13 that are used to define the liquid storage cavity 11; when the water tank 10 is mounted into the installation space 21, the second bottom wall 12 corresponds to the first bottom wall 22, and the second side wall 13 corresponds to the first side wall 23. In the above arrangement, the water tank 10 can be supported and mounted. The first bottom wall 22 is configured to support the water tank 10, the connection structure may be arranged on the bottom walls 22, 12 or on the side walls 23, 13, preferably is arranged on the side walls 23, 13. To facilitate mounting the water tank 10 by rotation and realize a connection to the installation space 21 while the water tank 10 is mounted, the first bottom wall 22 comprises a downwardly recessed arc-shaped area 221, the second bottom wall 12 is formed in a downwardly protruding arc shape that matches the arc-shaped area 221, and the connection structure may be arranged on the second side wall 13 and the first side wall 23.

The second side wall 13 and the first side wall 23 may have shapes that match each other respectively; for example, both the second side wall 13 and the first side wall 23 may be flat surfaces or curved surfaces, and may be complementary to each other in shape. In the case that the second side wall 13 and the first side wall 23 are complementary to each other in shape, for example, the second side wall 13 and the first side wall 23 have a plurality of bulges and recesses that match each other, the water tank 10 can be removably mounted in the installation space 21 by means of the complementary shapes of the second side wall 13 and the first side wall 23; in such a case, the connection structure comprises a plurality of matching bulges and recesses formed on the second side wall 13 and the first side wall 23 respectively. In the case that both the second side wall 13 and the first side wall 23 are flat surfaces or curved surfaces, matching connection components may be arranged on the second side wall 13 and the first side wall 23 to realize detachable installation.

Specifically, the connection structure may comprise a first connection component and a second connection component that are fitted with each other, the first connection component is arranged on one of the first side wall 23 and the second side wall 13, and the second connection component is arranged on the other of the first side wall 23 and the second side wall 13.

In order to improve the stability, reliability and flexibility of the installation of the water tank 10, the first connection component is on an upper part of the first side wall 23 or the second side wall 13, and the second connection component is on an upper part of the first side wall 23 or the second side wall 13. That is to say, the first connection component and the second connection component are spaced away from the second bottom wall 12 and the first bottom wall 22.

In the present disclosure, the first connection component and the second connection component may be any component or structure that can realize a detachable connection. For example, the first connection component and the second connection component may be magnetic components that attract each other.

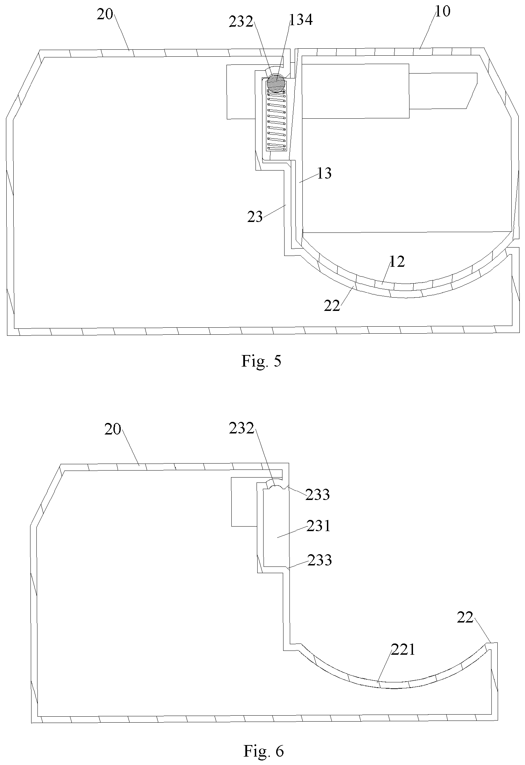

Specifically, according to an embodiment of the present disclosure, as shown in Figs. 2 - 6, the first connection component is arranged on an upper part of the first side wall 23, and the second connection component is arranged on an upper part of the second side wall 13. The first connection component comprises a recessed cavity 231 outwardly recessed (i.e., in the leftward direction in Fig. 5) from the inner wall surface of the first side wall 23 and a snap-fit groove 232 arranged on the inner surface of the recessed cavity 231, the second connection component comprises a boss 131 outwardly protruding (i.e., in the leftward direction in Fig. 5) from the second side wall 13 and a snap-fit piece 134 arranged on the boss 131, the boss 131 matches the recessed cavity 231, the snap-fit piece 134 matches the snap-fit groove 232, the snap-fit piece 134 is configured to be snap-fitted in the snap-fit groove 232 when the boss 131 is received in the recessed cavity 231, and can be detached from the snap-fit groove 232 when the boss 131 is subjected to an outwardly pulling force (i.e., a force for rotating the water tank 10 out of the installation space 21).

The snap-fit piece 134 may be snap-fitted in the snap-fit groove 232 and detached from the snap-fit groove 232 by means of its deformation; for example, the snap-fit piece 134 may be a protrusion formed on the boss 131 in a protruding manner. In order to improve the reliability of the fitting between the snap-fit piece 134 and the snap-fit groove 232 and prolong the service life of the snap-fit piece 134 and the snap-fit groove 232, preferably the snap-fit piece 134 is arranged to be snap-fitted with the snap-fit groove 232 and detached from the snap-fit groove 232 by movement.

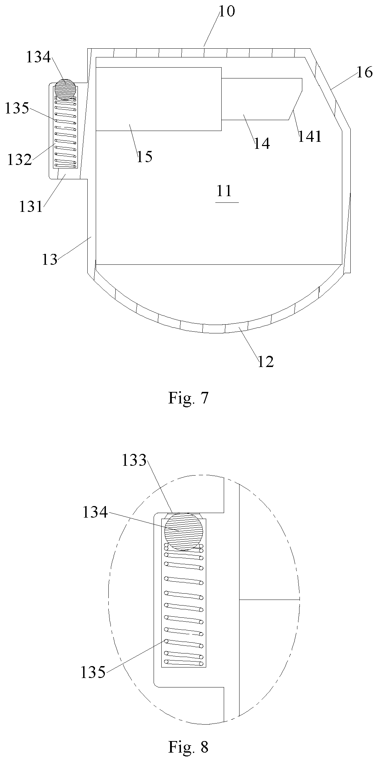

Specifically, in the embodiment shown in Figs. 2 - 8, the second connection component further comprises a compression spring 135, the boss 131 has a cavity 132 for receiving the compression spring 135 and the snap-fit piece 134, and has a hole 133 in communication with the cavity 132; the snap-fit piece 134 is configured to at least partially extend out of the hole 133 (as shown in Fig. 7) under an abutting action of the compression spring 135, and can retract into the cavity 132 (as shown in Fig. 8) by compressing the compression spring 135 under pressure. That is to say, the snap-fit piece 134 has a first state in which it at least partially extends out of the hole 133 and a second state in which it retracts into the cavity 132; in the first state, the snap-fit piece 134 can be snap-fitted in the snap-fit groove 232; in the second state, the snap-fit piece 134 can make way for the boss 131 to enter or exit the recessed cavity 231.

To facilitate the boss 131 to enter or exit the recessed cavity 231 quickly and successfully and further improve the convenience in the mounting and removal of the water tank 10, as shown in Fig. 6, two guiding bevels 233 may be arranged on the right upper and lower ends of the recessed cavity 231, so as to guide the boss 131 to enter or exit the recessed cavity 231.

In addition, as shown in Fig. 7, preferably the compression spring 135 is arranged in the vertical direction, the snap-fit piece 134 is located above the compression spring 135, the hole 133 is arranged at the top of the boss 131, and the snap-fit groove 232 is arranged at the top of the recessed cavity 231.

During the mounting, when the water tank 10 is loaded into the installation space 21 by rotation and the boss 131 is pushed into the recessed cavity 231, the guiding bevel 233 squeezes the snap-fit piece 134 to move downward to retract into the cavity 132 while it guides the boss 131 to enter the recessed cavity 231, till the boss 131 enters into the recessed cavity 231 entirely. At that point, the snap-fit piece 134 at least partially extends out of the hole 133 under the action of the compression spring 135 and thereby is snap-fitted in the snap-fit groove 232.

To ensure that the snap-fit piece 134 can extend and retract at the hole 133 successfully, preferably hole 133 is sized to be smaller than the snap-fit piece 134, which is to way, the snap-fit piece 134 can't extend out of the hole 133 entirely; instead, the snap-fit piece 134 partially extends out of the hole 133 under an abutting action of the compression spring 135.

As shown in Figs. 6 and 7, the snap-fit piece 134 may be a sphere, and the snap-fit groove 232 may be a hemispherical groove. In such a case, the hole 133 is a circular hole, the diameter of the hole 133 is smaller than the diameter of the snap-fit piece 134, and the size of the snap-fit groove 232 matches the size of the part of the snap-fit piece 134 that can extend out of the hole 133.

In the present disclosure, the connection structure may comprise a plurality of groups of first connection components and second connection components that match each other, so as to improve the stability and reliability of the installation of the water tank. Preferably, as shown in Fig. 2, the connection structure comprises two groups of first connection components and second connection components that match each other and arranged at an interval in the front-back direction.

In the present disclosure, as shown in Figs. 6 and 7, by configuring the first bottom wall 22 to comprise a downwardly recessed arc-shaped area 221 and configuring the second bottom wall 12 to form into a downwardly protruding arc-shape that matches the arc-shaped area 221, the supporting area for the water tank 10 can be increased, and the position of the water tank 10 can be limited. That is to say, the second bottom wall 12 can't be detached from the installation space 21 by moving it rightward horizontally with respect to the arc-shaped area 221. In addition, in the case that the connection structure comprises two groups of first connection components and second connection components that match each other, in association with the arc-shaped area 221 and the second bottom wall 12 that are fitted with each other, three-point fitting between the water tank 10 and the installation space 21 can be realized, thereby the stability of the water tank 10 is ensured.

The arc-shaped area 221 may be configured as a heating area; for example, the first bottom wall 22 may be formed from a heating plate, so that large-area heating of the water tank 10 can be realized.

As shown in Fig. 6, the axial direction of the arc-shaped area 221 (the front-back direction in Fig. 1) may be perpendicular to the mounting direction of the water tank 10 (the left-right direction in Fig. 1, i.e., the mounting direction of the boss 131 and the recessed cavity 231). In such a case, the water tank 10 can rotate around the axis of the arc-shaped area 221 within a certain range with respect to the arc-shaped area 221, so as to realize mounting and removal by rotation, and the angle of the water tank 10 can be adjusted conveniently to make the boss 131 and the recessed cavity 231 aligned to each other when the water tank 10 is mounted.

As shown in Fig. 5, to mount the water tank 10, the water tank 10 is placed on the arc-shaped area 221 first, with the second bottom wall 12 attached to the arc-shaped area 221, then the water tank 10 is rotated by a certain angle and pushed leftward to get close to the first side wall 23, so that the boss 131 enters the recessed cavity 231, and the snap-fit piece 134 is snap-fitted in the snap-fit groove 232; thus, the water tank 10 is mounted. To remove the water tank 10, the water tank 10 is directly pulled rightward, so that the snap-fit piece 134 moves downward and retracts into the cavity 132 under a squeezing action at the right side of the snap-fit groove 232, and the boss 131 is detached from the recessed cavity 231; thus, the water tank 10 can be removed.

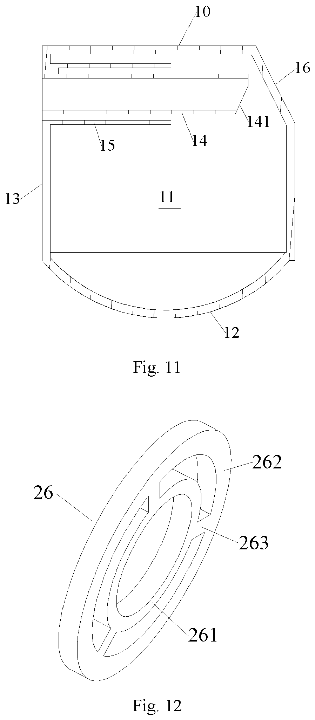

In the present disclosure, as shown in Figs. 7 and 11, the water tank 10 may comprise a second air inlet tube 14 and a second air outlet tube 15, wherein the second air inlet tube 14 is coaxially inserted in the second air outlet tube 15 and spaced from the second air outlet tube 15 by certain radial spacing to form a second air outflow channel, one end port of the second air inlet tube 14 and the second air outlet tube 15 (i.e., the port of the left end in Fig. 11) is on the second side wall 13, and the other end port of the second air inlet tube 14 and the second air outlet tube 15 (i.e., the port of the right end in Fig. 11) extends into the liquid storage cavity 11.

By configuring the second air inlet tube 14 and the second air outlet tube 15 into a nested structural form, not only the structure of the water tank can be simplified and the space in the liquid storage cavity can be saved, but also the disturbances to the air flow can be reduced; moreover, the annular second air outflow channel can condition the air flow naturally to avoid turbulence. The second air inlet tube 14 and the second air outlet tube 15 preferably are straight tube, and the second air inlet tube 14 can also serve as a water inlet tube to fill water into the water tank 10.

Preferably, the left end port of the second air inlet tube 14 and the second air outlet tube 15 is arranged on an upper part of the second side wall 13 and at a middle position of the second side wall 13 in the front-back direction, thus, the water in the water tank 10 will not flow back into the second air inlet tube 14 and the second air outlet tube 15 when the water tank 10 is tilted as a result of handling or movement, etc.

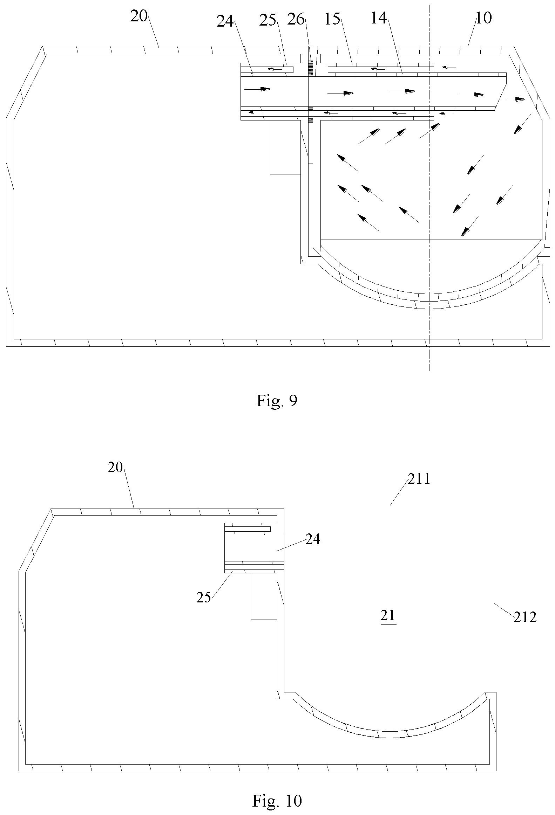

As shown in Fig. 9, preferably, the length of the second air inlet tube 14 is greater than that of the second air outlet tube 15, the right end port of the second air outlet tube 15 is at the right side of the center line of the water tank (indicated by the dotted line in Fig. 9), the right end port of the second air inlet tube 14 is formed as an inclined port 141, and the wall of the water tank 10 opposite to the inclined port 141 is formed as an inclined wall 16, so that the air from the inclined port 141 is deflected toward a lower middle area of the liquid storage cavity 11.

As indicated by the arrow in Fig. 9, after the air flows out of the second air inlet tube 14, it is deflected by the inclined wall 16 to flow toward the water surface direction, then is deflected by the water surface and other side walls of the water tank 10 for several times and enters into the second air outflow channel, and finally flows out of the second air outflow channel. The air carries warm and humid moisture content in the entire flow process, thereby a good air humidifying effect is attained. Moreover, as shown in Fig. 9, the water tank 10 has a simple internal structure without any useless part, and the air flow simply undergoes several times of deflection inside the water tank, without any turbulence.

To realize the fitting between the second air inlet tube 14 and the second air outlet tube 15, as shown in Figs. 9 - 11, the water tank installation structure may further comprise a first air inlet tube 24 and a first air outlet tube 25, wherein the first air inlet tube 24 is coaxially inserted in the first air outlet tube 25 and spaced from the first air outlet tube 25 by certain radial spacing to form a first air outflow channel, one end port (i.e., the right end port in Fig. 9) of the first air inlet tube 24 and the first air outlet tube 25 is on the first side wall 23, and the right end port of the first air inlet tube 24 and the first air outlet tube 25 may be communicated with the left end port of the second air inlet tube 14 and the second air outlet tube 15 in a hermetically sealed manner via a sealing element 26. It can be understood that the first air inlet tube 24 is communicated with the second air inlet tube 14, and the first air outlet tube 25 is communicated with the second air outlet tube 15 (i.e., the first air outflow channel is communicated with the second air outflow channel).

As shown in Fig. 12, the sealing element 26 is an annular member comprising an inner ring 261 and an outer ring 262 that are nested coaxially, the inner cavity of the inner ring 261 is communicated with the first air inlet tube 24 and the second air inlet tube 14, and there is certain radial spacing between the inner ring 261 and the outer ring 262 to form a communication channel for the first air outflow channel and the second air outflow channel to be communicated with each other.

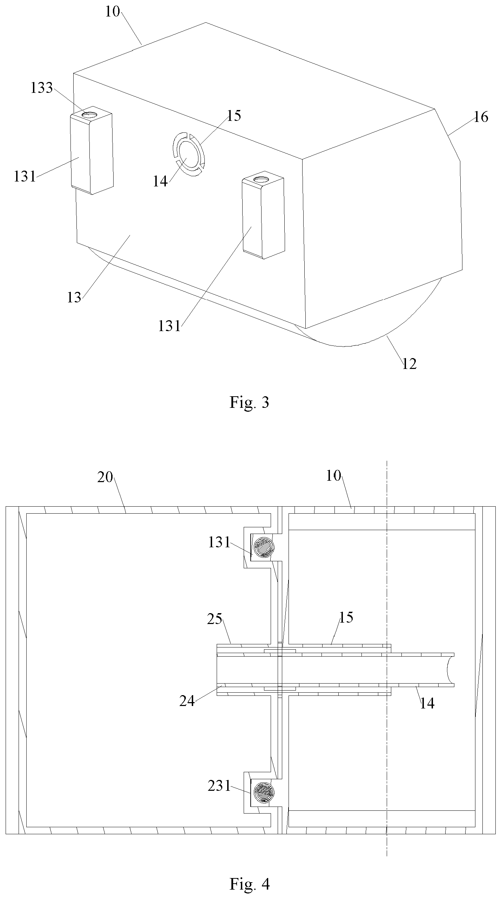

In addition, as shown in Fig. 12, the inner ring 261 and the outer ring 262 may be connected to each other via a plurality of connection ribs 263 that are spaced apart from each other. With the arrangement of the connection ribs 263, the communication channel can be separated into a plurality of arc-shaped channels, so as to attain an air flow conditioning effect. Accordingly, as shown in Figs. 2 and 3, the second air inlet tube 14 may be connected with the second air outlet tube 15 and the first air inlet tube 24 may be connected with the first air outlet tube 25 via connecting members respectively, so that the second air outflow channel and the first air outflow channel are separated into a plurality of arc-shaped channels respectively, which correspond to the arc-shaped channels of the sealing element 26, so as to condition the out-flowing air effectively, thereby reduce pneumatic noises.

The sealing element 26 may be made of a sealing material, such as rubber, silica gel, or the like.

With the above arrangement, not only the structure can be simplified and the space can be saved, but also the disturbances to the air flow can be reduced; in addition, the annular air outflow channel can condition the air flow naturally and avoid turbulence. Moreover, since the air inlet tubes and the air outlet tubes are in a built-in form, the ports can be butt-jointed and communicated by using the sealing element 26, so that the water tank 10 can be mounted and removed independently from the air inlet tubes and the air outlet tubes. Therefore, the water tank can be mounted and removed more conveniently, and the water tank installation structure has smaller space occupation. Thus, when the water tank installation structure is applied in a ventilation therapy device, the space occupation of the ventilation therapy device can be decreased, and the esthetic appearance of the ventilation therapy device can be improved.

With the above water tank installation structure provided by the present disclosure, the user can mount and removed the water tank 10 by one-handed operation, and the water tank can be mounted, removed and filled water conveniently, thereby a good experience is achieved; the above water tank installation structure has further advantages including high safety and high reliability, simple structure, low failure rate, less parts, low cost, stable air flow, and high patient compliance, etc.

In another aspect, the present disclosure provides a ventilation therapy device, which comprises a main machine 20 and the water tank installation structure described above, wherein the installation space 21 is arranged on the main machine 20.

Specifically, as shown in Figs. 1 and 2, the installation space 21 is arranged on the right side of the main machine 20, a first side wall 23 and a first bottom wall 22 for defining an installation space 21 are arranged on the main machine 20, the top surface, front surface and back surface of the water tank 10 are flush with corresponding sides of the main machine 20 after the water tank 10 is mounted in the installation space 21, thereby a higher esthetic appearance of the ventilation therapy device is achieved.

In addition, it should be noted that a fan is mounted in the left part of the main machine 20, the first air inlet tube 24 is communicated with an air outlet of the fan, and an air outlet communicated with the first air outlet tube 25 is arranged on the main machine 20. During use, the fan can draw ambient air into the main machine 20, then the air is fed into the first air inlet tube 24, and flows through the second air inlet tube 14 into the liquid storage cavity 11 of the water tank 10, wherein the air is humidified; then the air is discharged from the main machine through the second air outflow channel, the first air outflow channel and the air outlet sequentially, and then is delivered to the patient side for the patient to inhale.

In view that the main improvements in the present disclosure lie in the water tank and the installation of the water tank, and the arrangement of a fan inside the main machine belongs to a known technique in the art, the fan and the installation of the fan are not detailed here.

In the present disclosure, the ventilation therapy device may be a ventilator or an oxygen therapy apparatus, or the like.

The scope of the present invention is defined by the following appended claims.

## Claims

1. A water tank installation structure for a ventilation therapy device, comprising a water tank (10), an installation space (21), and a connection structure, wherein the water tank (10) is configured to fit into and out of the installation space (21) in a rotational manner, the connection structure is configured to connect the water tank (10) to the installation space (21) when the water tank (10) is rotated into the installation space (21), and release the connection of the water tank (10) from the installation space (21) when the water tank (10) is detached from the installation space (21);
wherein the water tank installation structure comprises a first bottom wall (22), and a first side wall (23) which define the installation space (21), the water tank (10) comprises a liquid storage cavity (11), and a second bottom wall (12) and a second side wall (13) that define the liquid storage cavity (11); the second bottom wall (12) corresponds to the first bottom wall (22) and the second side wall (13) corresponds to the first side wall (23) when the water tank (10) is mounted into the installation space (21),
**characterized in that**
the first bottom wall (22) comprises a downwardly recessed arc-shaped area (221), the second bottom wall (12) forms a downwardly protruding arc-shape that matches the arc-shaped area (221).

2. The water tank installation structure for a ventilation therapy device of claim 1, wherein the installation space (21) has a top opening (211) and a side opening (212), which are used for mounting the water tank (10) into the installation space (21).

3. The water tank installation structure for a ventilation therapy device of claim 2 or 3, wherein the connection structure comprises a first connection component and a second connection component that are fitted with each other, the first connection component is arranged on one of the first side wall (23) and the second side wall (13), and the second connection component is arranged on the other of the first side wall (23) and the second side wall (13).

4. The water tank installation structure for a ventilation therapy device of claim 3, wherein the first connection component is on an upper part of the first side wall (23) or the second side wall (13), and the second connection component is on an upper part of the first side wall (23) or the second side wall (13).

5. The water tank installation structure for a ventilation therapy device of claim 3 or 4, wherein the first connection component is arranged on the first side wall (23), the second connection component is arranged on the second side wall (13), the first connection component comprises a recessed cavity (231) outwardly recessed from an inner wall surface of the first side wall (23) and a snap-fit groove (232) arranged on an inner surface of the recessed cavity (231), the second connection component comprises a boss (131) outwardly protruding from an outer wall surface of the second side wall (13) and a snap-fit piece (134) arranged on the boss (131), the boss (131) matches the recessed cavity (231), the snap-fit piece (134) matches the snap-fit groove (232), the snap-fit piece (134) is arranged to be snap-fitted in the snap-fit groove (232) when the boss (131) is received in the recessed cavity (231), and is able to be detached from the snap-fit groove (232) when the boss (131) is subjected to an outwardly pulling force.

6. The water tank installation structure for a ventilation therapy device of claim 5, wherein the second connection component comprises a compression spring (135), the boss (131) has a cavity (132) therein for accommodating the compression spring (135) and the snap-fit piece (134), the boss (131) has a hole (133) in communication with the cavity (132), the snap-fit piece (134) is configured to at least partially extend out of the hole (133) under an abutting action of the compression spring (135), and is able to retract into the cavity (132) by compressing the compression spring (135) under pressure.

7. The water tank installation structure for a ventilation therapy device of claim 6, wherein the size of the hole (133) is smaller than the size of the snap-fit piece (134).

8. The water tank installation structure for a ventilation therapy device of claim 6, wherein the snap-fit piece (134) is a sphere, and the snap-fit groove (232) is a hemispherical groove.

9. The water tank installation structure for a ventilation therapy device of claim 1, wherein the arc-shaped area (221) is a heating area for heating the water tank (10).

10. The water tank installation structure for a ventilation therapy device of any of claims 1 - 9, wherein the water tank installation structure comprises a first air inlet tube (24), a first air outlet tube (25), a second air inlet tube (14) and a second air outlet tube (15), the first air inlet tube (24) is coaxially inserted in the first air outlet tube (25) and spaced from the first air outlet tube (25) by certain radial spacing to form a first air outflow channel, and one end port of the first air inlet tube (24) and the first air outlet tube (25) is located on the first side wall (23); the second air inlet tube (14) is coaxially inserted in the second air outlet tube (15) and spaced from the second air outlet tube (15) by certain radial spacing to form a second air outflow channel, one end port of the second air inlet tube (14) and the second air outlet tube (15) is located on the second side wall (13), and the other end port of the second air inlet tube (14) and the second air outlet tube (15) extends into the liquid storage cavity (11), and the one end port of the first air inlet tube (24) and the first air outlet tube (25) is communicated with the one end port of the second air inlet tube (14) and the second air outlet tube (15) in a hermetically sealed manner via a sealing element (26).

11. The water tank installation structure for a ventilation therapy device of claim 10, wherein the one end port of the second air inlet tube (14) and the second air outlet tube (15) is located on the upper part of the second side wall (13).

12. The water tank installation structure for a ventilation therapy device of claim 10, wherein the length of the second air inlet tube (14) is greater than the length of the second air outlet tube (15), the other end port of the second air inlet tube (14) is formed as an inclined port (141), a wall of the water tank (10) opposite to the inclined port (141) is formed as an inclined wall (16), so that the air from the inclined port (141) is deflected toward a lower middle area of the liquid storage cavity (11).

13. A ventilation therapy device, comprising a main machine (20) and the water tank installation structure of any of claims 1 - 12, wherein the installation space (21) is arranged on the main machine (20).

## Patentansprüche

1. Wassertank-Installationsstruktur für eine Beatmungsbehandlungs-Vorrichtung, die einen Wassertank (10), einen Installationsraum (21) sowie eine Verbindungsstruktur umfasst, wobei der Wassertank (10) so ausgeführt ist, dass er mittels Drehung in den Installationsraum (21) eingesetzt und aus ihm entnommen wird, die Verbindungsstruktur so ausgeführt ist, dass sie den Wassertank (10) mit dem Installationsraum (21) verbindet, wenn der Wassertank (10) in den Installationsraum (21) hinein gedreht wird und die Verbindung des Wassertanks (10) mit dem Installationsraum (21) löst, wenn der Wassertank (10) aus dem Installationsraum (21) entfernt wird;
wobei die Wassertank-Installationsstruktur eine erste untere Wand (22) und eine erste seitliche Wand (23) umfasst, die den Installationsraum (21) begrenzen, der Wassertank (10) einen Flüssigkeits-Speicherungshohlraum (11) umfasst, sowie eine zweite untere Wand (12) und eine zweite seitliche Wand (13) umfasst, die den Flüssigkeits-Speicherungshohlraum (11) begrenzen, wobei die zweite untere Wand (12) der ersten unteren Wand (22) entspricht und die zweite seitliche Wand (13) der ersten seitlichen Wand (23) entspricht, wenn der Wassertank (10) in den Installationsraum (21) eingebaut wird,
**dadurch gekennzeichnet, dass**
die erste untere Wand (22) einen nach unten vertieften bogenförmigen Bereich (221) umfasst und die zweite untere Wand (12) eine nach unten vorstehende Bogenform bildet, die dem bogenförmigen Bereich (221) entspricht.

2. Wassertank-Installationsstruktur für eine Beatmungsbehandlungs-Vorrichtung nach Anspruch 1, wobei der Installationsraum (21) eine obere Öffnung (211) sowie eine seitliche Öffnung (212) aufweist, die zum Einbauen des Wassertanks (10) in den Installationsraum (21) dienen.

3. Wassertank-Installationsstruktur für eine Beatmungsbehandlungs-Vorrichtung nach Anspruch 2 oder 3, wobei die Verbindungsstruktur eine erste Verbindungskomponente und eine zweite Verbindungskomponente umfasst, die ineinandergepasst werden, die erste Verbindungskomponente an der ersten seitlichen Wand (23) oder der zweiten seitlichen Wand (13) angeordnet ist und die zweite Verbindungskomponente an der anderen von der ersten seitlichen Wand (23) und der zweiten seitlichen Wand (13) angeordnet ist.

4. Wassertank-Installationsstruktur für eine Beatmungsbehandlungs-Vorrichtung nach Anspruch 3, wobei sich die erste Verbindungskomponente an einem oberen Teil der ersten seitlichen Wand (23) oder der zweiten seitlichen Wand (13) befindet und sich die zweite Verbindungskomponente an einem oberen Teil der ersten seitlichen Wand (23) oder der zweiten seitlichen Wand (13) befindet.

5. Wassertank-Installationsstruktur für eine Beatmungsbehandlungs-Vorrichtung nach Anspruch 3 oder 4, wobei die erste Verbindungskomponente an der ersten seitlichen Wand (23) angeordnet ist, die zweite Verbindungskomponente an der zweiten seitlichen Wand (13) angeordnet ist, und die erste Verbindungskomponente einen vertieften Hohlraum (231), der von einer inneren Wandfläche der ersten seitlichen Wand (23) nach außen hin vertieft ist, sowie eine Einrast-Nut (232) umfasst, die an einer inneren Fläche des vertieften Hohlraums (231) angeordnet ist, die zweite Verbindungskomponente einen Vorsprung (131), der von einer äußeren Wandfläche der zweiten seitlichen Wand (13) vorsteht, sowie ein Einrastteil (134) umfasst, das an dem Vorsprung (131) angeordnet ist, wobei der Vorsprung (131) in den vertieften Hohlraum (231) passt, das Einrastteil (134) so eingerichtet ist, dass es in die Einrast-Nut (232) einrastet, wenn der Vorsprung (131) in dem vertieften Hohlraum (231) aufgenommen wird, und aus der Einrast-Nut (232) entfernt werden kann, wenn der Vorsprung (131) einer nach außen gerichteten Ziehkraft ausgesetzt wird.

6. Wassertank-Installationsstruktur für eine Beatmungsbehandlungs-Vorrichtung nach Anspruch 5, wobei die zweite Verbindungskomponente eine Druckfeder (135) umfasst, in dem Vorsprung (131) einen Hohlraum (132) zum Aufnehmen der Druckfeder (135) sowie des Einrastteils (134) aufweist, der Vorsprung (131) ein Loch (133) aufweist, das mit dem Hohlraum (132) in Verbindung steht, das Einrastteil (134) so ausgeführt ist, dass es sich durch Anliegen an der Druckfeder (135) wenigstens teilweise aus dem Loch (133) nach außen erstreckt, und es durch Zusammendrücken der Druckfeder (135) unter Druck in den Hohlraum (132) einfahren kann.

7. Wassertank-Installationsstruktur für eine Beatmungsbehandlungs-Vorrichtung nach Anspruch 6, wobei die Größe des Lochs (133) geringer ist als die Größe des Einrastteils (134).

8. Wassertank-Installationsstruktur für eine Beatmungsbehandlungs-Vorrichtung nach Anspruch 6, wobei das Einrastteil (134) eine Kugel ist und die Einrast-Nut (232) eine halbkugelförmige Nut ist.

9. Wassertank-Installationsstruktur für eine Beatmungsbehandlungs-Vorrichtung nach Anspruch 1, wobei der bogenförmige Bereich (221) ein Aufheizbereich zum Aufheizen des Wassertanks (10) ist.

10. Wassertank-Installationsstruktur für eine Beatmungsbehandlungs-Vorrichtung nach einem der Ansprüche 1 - 9, wobei die Wassertank-Installationsstruktur eine erste Luft-Einlassröhre (24), eine erste Luft-Auslassröhre (25) eine zweite Luft-Einlassröhre (14) sowie eine zweite Luft-Auslassröhre (15) umfasst, die erste Luft-Einlassröhre (24) koaxial in die erste Luft-Auslassröhre (25) eingeführt und von der ersten Luft-Auslassröhre (25) um einen bestimmten radialen Zwischenraum beabstandet ist und so einen ersten Luft-Ausströmkanal bildet, und sich ein Endanschluss der ersten Luft-Einlassröhre (24) sowie der ersten Luft-Auslassröhre (25) an der ersten seitlichen Wand (23) befindet; die zweite Luft-Einlassröhre (14) koaxial in die zweite Luft-Auslassröhre (15) eingeführt und von der zweiten Luft-Auslassröhre (15) um einen bestimmten radialen Zwischenraum beabstandet ist und so einen zweiten Luft-Ausströmkanal bildet, sich ein Endanschluss der zweiten Luft-Einlassröhre (14) sowie der zweiten Luft-Auslassröhre (15) an der zweiten seitlichen Wand (13) befindet, und sich der andere Endanschluss der zweiten Luft-Einlassröhre (14) sowie der zweiten Luft-Auslassröhre (25) in den Flüssigkeits-Speicherungshohlraum (11) hinein erstreckt und der eine Endanschluss der ersten Luft-Einlassröhre (24) sowie der ersten Luft-Auslassröhre (25) mit dem einen Endanschluss der zweiten Luft-Einlassröhre (14) sowie der zweiten Luft-Auslassröhre (15) über ein Dichtungselement (26) hermetisch abgedichtet in Verbindung steht.

11. Wassertank-Installationsstruktur für eine Beatmungsbehandlungs-Vorrichtung nach Anspruch 10, wobei sich der eine Endanschluss der zweiten Luft-Einlassröhre (14) sowie der zweiten Luft-Auslassröhre (15) an dem oberen Teil der zweiten seitlichen Wand (13) befindet.

12. Wassertank-Installationsstruktur für eine Beatmungsbehandlungs-Vorrichtung nach Anspruch 10, wobei die Länge der zweiten Luft-Einlassröhre (14) größer ist als die Länge der zweiten Luft-Auslassröhre (15), der andere Endanschluss der zweiten Luft-Einlassröhre (14) als ein schräger Anschluss (141) ausgebildet ist und eine Wand des Wassertanks (10), gegenüber dem schrägen Anschluss (141) als eine schräge Wand (16) ausgebildet ist, so dass die Luft von dem schrägen Anschluss (141) zu einem unteren Mittelbereich des Flüssigkeits-Speicherungshohlraums (11) hin abgelenkt wird.

13. Beatmungsbehandlungs-Vorrichtung, die ein Hauptaggregat (20) sowie die Wassertank-Installationsstruktur nach einem der Ansprüche 1 - 12 umfasst, wobei der Installationsraum (21) an dem Hauptaggregat (20) angeordnet ist.

## Revendications

1. Structure d'installation d'une cuve d'eau pour un dispositif de thérapie par ventilation, comprenant une cuve d'eau (10), un espace d'installation (21), et une structure de raccordement, dans laquelle la cuve d'eau (10) est configurée pour s'ajuster dans, et hors, de l'espace d'installation (21) d'une manière rotative, la structure de raccordement est configurée pour raccorder la cuve d'eau (10) à l'espace d'installation (21) lorsque la cuve d'eau (10) est tournée dans l'espace d'installation (21), et pour libérer le raccordement de la cuve d'eau (10) de l'espace d'installation (21) lorsque la cuve d'eau (10) est détachée de l'espace d'installation (21) ;
dans laquelle la structure d'installation d'une cuve d'eau comprend une première paroi inférieure (22), et une première paroi latérale (23) qui définissent l'espace d'installation (21), la cuve d'eau (10) comprend une cavité de stockage de liquide (11), et une seconde paroi inférieure (12) et une seconde paroi latérale (13) qui définissent la cavité de stockage de liquide (11) ; la seconde paroi inférieure (12) correspond à la première paroi inférieure (22) et la seconde paroi latérale (13) correspond à la première paroi latérale (23) lorsque la cuve d'eau (10) est montée dans l'espace d'installation (21),
**caractérisée en ce que**
la première paroi inférieure (22) comprend une surface en forme d'arc (221), renfoncée vers le bas, la seconde paroi inférieure (12) présente une forme d'arc faisant saillie vers le bas qui correspond à la surface en forme d'arc (221).

2. Structure d'installation d'une cuve d'eau pour un dispositif de thérapie par ventilation selon la revendication 1, dans laquelle l'espace d'installation (21) présente une ouverture supérieure (211) et une ouverture latérale (212), qui sont utilisées pour monter la cuve d'eau (10) dans l'espace d'installation (21).

3. Structure d'installation d'une cuve d'eau pour un dispositif de thérapie par ventilation selon la revendication 2 ou 3, dans laquelle la structure de raccordement comprend un premier composant de raccordement et un second composant de raccordement qui sont ajustés l'un par rapport à l'autre, le premier composant de raccordement est agencé sur l'une de la première paroi latérale (23) et de la seconde paroi latérale (13), et le second composant de raccordement est agencé sur l'autre de la première paroi latérale (23) et de la seconde paroi latérale (13).

4. Structure d'installation d'une cuve d'eau pour un dispositif de thérapie par ventilation selon la revendication 3, dans laquelle le premier composant de raccordement se trouve sur une partie supérieure de la première paroi latérale (23) ou de la seconde paroi latérale (13), et le second composant de raccordement se trouve sur une partie supérieure de la première paroi latérale (23) ou de la seconde paroi latérale (13).

5. Structure d'installation d'une cuve d'eau pour un dispositif de thérapie par ventilation selon la revendication 3 ou 4, dans laquelle le premier composant de raccordement est agencé sur la première paroi latérale (23), le second composant de raccordement est agencé sur la seconde paroi latérale (13), le premier composant de raccordement comprend une cavité renfoncée (231) renfoncée vers l'extérieur depuis une surface de paroi interne de la première paroi latérale (23) et une rainure d'ajustement par pression (232) agencée sur une surface interne de la cavité renfoncée (231), le second composant de raccordement comprend une bosse (131) faisant saillie vers l'extérieur depuis une surface de paroi externe de la seconde paroi latérale (13) et un élément d'ajustement par pression (134) agencé sur la bosse (131), la bosse (131) correspond à la cavité renfoncée (231), l'élément d'ajustement par pression (134) correspond à la rainure d'ajustement par pression (232), l'élément d'ajustement par pression (134) est agencé pour être ajusté par pression dans la rainure d'ajustement par pression (232) lorsque la bosse (131) est reçue dans la cavité renfoncée (231), et est capable d'être détachée de la rainure d'ajustement par pression (232) lorsque la bosse (131) est soumise à une force de traction vers l'extérieur.

6. Structure d'installation d'une cuve d'eau pour un dispositif de thérapie par ventilation selon la revendication 5, dans laquelle le second composant de raccordement comprend un ressort de compression (135), la bosse (131) présente une cavité (132) à l'intérieur pour loger le ressort de compression (135) et l'élément d'ajustement par pression (134), la bosse (131) présente un trou (133) en communication avec la cavité (132), l'élément d'ajustement par pression (134) est configuré pour s'étendre au moins partiellement hors du trou (133) sous une action de mise en butée du ressort de compression (135), et est capable de se rétracter dans la cavité (132) en comprimant le ressort de compression (135) sous pression.

7. Structure d'installation d'une cuve d'eau pour un dispositif de thérapie par ventilation selon la revendication 6, dans laquelle la taille du trou (133) est inférieure à la taille de l'élément d'ajustement par pression (134).

8. Structure d'installation d'une cuve d'eau pour un dispositif de thérapie par ventilation selon la revendication 6, dans laquelle l'élément d'ajustement par pression (134) est une sphère, et la rainure d'ajustement par pression (232) est une rainure hémisphérique.

9. Structure d'installation d'une cuve d'eau pour un dispositif de thérapie par ventilation selon la revendication 1, dans laquelle la surface en forme d'arc (221) est une surface de chauffage pour chauffer la cuve d'eau (10).

10. Structure d'installation d'une cuve d'eau pour un dispositif de thérapie par ventilation selon l'une quelconque des revendications 1 à 9, dans laquelle la structure d'installation d'une cuve d'eau comprend un premier tube d'entrée d'air (24), un premier tube de sortie d'air (25), un second tube d'entrée d'air (14) et un second tube de sortie d'air (15), le premier tube d'entrée d'air (24) est coaxialement inséré dans le premier tube de sortie d'air (25) et espacé du premier tube de sortie d'air (25) d'un certain espacement radial pour former un premier canal de sortie d'écoulement d'air, et un orifice d'extrémité du premier tube d'entrée d'air (24) et du premier tube de sortie d'air (25) est localisé sur la première paroi latérale (23) ; le second tube d'entrée d'air (14) est coaxialement inséré dans le second tube de sortie d'air (15) et espacé du second tube de sortie d'air (15) d'un certain espacement radial pour former un second canal de sortie d'écoulement d'air, un orifice d'extrémité du second tube d'entrée d'air (14) et du second tube de sortie d'air (15) est localisé sur la seconde paroi latérale (13), et l'autre orifice d'extrémité du second tube d'entrée d'air (14) et du second tube de sortie d'air (15) s'étend à l'intérieur de la cavité de stockage de liquide (11), et le seul orifice d'extrémité du premier tube d'entrée d'air (24) et du premier tube de sortie d'air (25) communique avec le seul orifice d'extrémité du second tube d'entrée d'air (14) et du second tube de sortie d'air (15) d'une manière hermétiquement scellée par l'intermédiaire d'un élément d'étanchéité (26).

11. Structure d'installation d'une cuve d'eau pour un dispositif de thérapie par ventilation selon la revendication 10, dans laquelle l'orifice d'extrémité du second tube d'entrée d'air (14) et du second tube de sortie d'air (15) est localisé sur la partie supérieure de la seconde paroi latérale (13).

12. Structure d'installation d'une cuve d'eau pour un dispositif de thérapie par ventilation selon la revendication 10, dans laquelle la longueur du second tube d'entrée d'air (14) est supérieure à la longueur du second tube de sortie d'air (15), l'autre orifice d'extrémité du second tube d'entrée d'air (14) se présente sous la forme d'un orifice incliné (141), une paroi de la cuve d'eau (10) opposée à l'orifice incliné (141) se présente sous la forme d'une paroi inclinée (16), de sorte que l'air provenant de l'orifice incliné (141) est dévié vers une zone médiane inférieure de la cavité de stockage de liquide (11).

13. Dispositif de thérapie par ventilation, comprenant une machine principale (20) et la structure d'installation d'une cuve d'eau selon l'une quelconque des revendications 1 à 12, dans lequel l'espace d'installation (21) est agencé sur la machine principale (20).
